# DEMANDE DE BREVET EUROPEEN

(11) **EP 4 755 314 A1**
(43) Date de publication de la demande: **10.06.2026**
(21) Numéro de dépôt: 25217159.0
(22) Date de dépôt: 20.11.2025
(51) Int. Cl.: A61B 8/12, A61B 8/00

(54) **GAINE D'INTRODUCTION MEDICALE MUNIE D'UNE SONDE D'IMAGERIE ULTRASONORE**

(30) Priorité: 05.12.2024 FR 2413507
(71) Demandeur: Vermon, 37000 Tours (FR)
(72) Inventeur: MATEO, Tony, 37000 TOURS (FR); FERIN, Guillaume, 37000 TOURS (FR); CHEPPE, Maxime, 37000 TOURS (FR)
(74) Mandataire: Cabinet Beaumont

(57) **Abrégé**

La présente description concerne une gaine d'introduction médicale (200) s'étendant selon une direction axiale (X) entre une extrémité distale (200A) et une extrémité proximale (200B), et comportant une lumière axiale (204) et une portion fléchissable (210) s'étendant jusqu'à l'extrémité distale et comprenant :
- une sonde d'imagerie ultrasonore (220) comprenant plusieurs transducteurs à ultrasons répartis en matrice sur une face transversale (200C) de la gaine d'introduction médicale à l'extrémité distale autour de la lumière axiale ;
- un coupleur d'orientation (211) adapté à conférer une courbure à la portion fléchissable ;
- un fourreau (215) disposé autour du coupleur d'orientation et comprenant une portion tubulaire (216) et une portion périphérique (217) protubérante enroulée en plusieurs tours en saillie (219) autour de la portion tubulaire le long de la direction axiale ; et
- des bandes de connexion (231) s'étendant depuis les transducteurs dans la direction axiale en direction de l'extrémité proximale et réparties autour du fourreau ; et
- une structure élastique (218) enroulée en plusieurs tours autour du fourreau et des bandes de connexion, la structure élastique cheminant entre les tours en saillie.

## Description

### Domaine technique

La présente description concerne de façon générale les dispositifs d'exploration et d'intervention intracorporels destinés à être introduits, au moins partiellement, dans une région anatomique d'un patient, et munis d'une sonde d'imagerie ultrasonore pouvant être dirigée vers la région anatomique du patient.

La présente description concerne en particulier une gaine d'introduction médicale, par exemple un trocart ou une canule, munie d'une sonde d'imagerie ultrasonore intégrée à une extrémité distale de la gaine d'introduction médicale.

La gaine d'introduction médicale comprend par exemple une lumière interne adaptée à recevoir un cathéter, ou tout autre appareil médical allongé.

### Technique antérieure

Certaines techniques d'exploration et d'intervention dans des régions anatomiques, telles que des régions cardiaques, utilisent de l'imagerie, par exemple la radioscopie ou fluoroscopie (c'est-à-dire de l'imagerie par rayons X), pour aider à localiser et à guider des appareils médicaux jusqu'à une région cible du patient, généralement à travers la vascularisation du patient.

Les appareils médicaux pouvant être guidés dans le corps d'un patient comprennent typiquement des dispositifs d'accès, ou gaines d'introduction médicale, par exemple des trocarts ou des canules, des cathéters de diagnostic, et/ou des cathéters de traitement, par exemple des cathéters d'ablation ou de remplacement.

Des cathéters et des sondes d'imagerie ultrasonore ont été développés pour visualiser directement la région cible. Par exemple, un cathéter intégrant une sonde d'imagerie ultrasonore, ou cathéter d'imagerie, peut être utilisé pour imager la région cible, puis guider un dispositif d'accès et un cathéter de traitement dans cette région cible.

Cependant, généralement, le cathéter d'imagerie est distinct du dispositif d'accès et du cathéter de traitement. D'une part, cela nécessite de multiplier les opérations d'introduction/évacuation des différents appareils, voire le nombre d'accès, dans le corps du patient, avec les risques et inconforts que cela implique pour le patient, et impliquant de facto l'allongement du temps d'intervention. D'autre part, cela nécessite d'avoir un suivi de position pour suivre l'emplacement de chaque appareil dans le corps du patient, ce qui est généralement réalisé par la fluoroscopie, ce qui peut multiplier l'exposition du patient, et du personnel médical, aux rayons X. Cela peut en outre nécessiter de faire des recoupements de positionnements entre les différents appareils, ce qui peut être une source de perte de temps additionnelle.

Il serait souhaitable de pouvoir disposer d'un dispositif d'exploration et d'intervention palliant au moins en partie certains des inconvénients des dispositifs d'exploration et d'intervention intracorporels connus.

En particulier, il existe un besoin pour une gaine d'introduction médicale, qui soit adaptée à recevoir un cathéter ou tout autre appareil médical allongé et qui comprenne une fonctionnalité d'imagerie. Il serait avantageux que la gaine d'introduction médicale puisse être aisément orientable, de manière à être guidée vers une région anatomique cible.

### Résumé de l'invention

Un mode de réalisation pallie tout ou partie des inconvénients des dispositifs d'exploration et d'intervention connus.

Un mode de réalisation prévoit une gaine d'introduction médicale s'étendant selon une direction axiale entre une extrémité distale et une extrémité proximale opposée à l'extrémité distale, la gaine d'introduction médicale comportant une lumière axiale interne entre ladite extrémité distale et ladite extrémité proximale, et une portion fléchissable s'étendant jusqu'à ladite extrémité distale, la portion fléchissable comprenant :
- une sonde d'imagerie ultrasonore comprenant plusieurs transducteurs à ultrasons répartis en matrice sur une face transversale de la gaine d'introduction médicale à l'extrémité distale, autour de la lumière axiale ;
- un coupleur d'orientation adapté à conférer une courbure à la portion fléchissable ;
- un fourreau disposé autour du coupleur d'orientation, ledit fourreau comprenant une portion tubulaire et une portion périphérique protubérante enroulée en plusieurs tours en saillie autour de la portion tubulaire, le long de la direction axiale, la portion tubulaire étant comprise entre la portion périphérique et le coupleur d'orientation ; et
- des bandes de connexion s'étendant depuis les transducteurs à ultrasons dans la direction axiale en direction de l'extrémité proximale et réparties autour du fourreau ; et
- une structure élastique enroulée en plusieurs tours autour du fourreau et des bandes de connexion, la structure élastique cheminant entre les tours en saillie.

Selon un mode de réalisation, la portion périphérique présente une forme en hélice, les tours en saillie correspondant aux tours de l'hélice, la structure élastique présentant également une forme en hélice cheminant entre les tours en saillie.

Selon un mode de réalisation, les tours en saillie de la portion périphérique sont des anneaux en saillie disjoints entre eux, et répartis, par exemple régulièrement répartis, sur plusieurs circonférences de la portion tubulaire le long de la direction axiale, les tours de la structure élastique étant également des anneaux disjoints entre eux et positionnés entre les anneaux en saillie.

Selon un mode de réalisation, le pas entre les tours de la structure élastique est sensiblement égal au pas entre les tours en saillie de la portion périphérique, les tours de la structure élastique étant répartis de manière régulière le long de la direction axiale.

Selon un mode de réalisation, le pas entre les tours de la structure élastique est sensiblement égal au pas entre les tours en saillie de la portion périphérique, les tours de la structure élastique étant répartis de manière irrégulière le long de la direction axiale, par exemple avec une plus grande densité autour d'un centre de la portion fléchissable.

Selon un mode de réalisation, la lumière axiale est dimensionnée pour recevoir de manière coulissante et/ou rotative un cathéter.

Selon un mode de réalisation, le coupleur d'orientation comprend des maillons, par exemple des rotules, deux maillons adjacents coopérant l'un avec l'autre de manière à pouvoir pivoter autour d'au moins un axe de rotation.

Selon un mode de réalisation, le coupleur d'orientation comprend des câbles de tringlerie reliés aux maillons de manière à maintenir les maillons les uns contre les autres, et à contrôler les pivotements des maillons, et ainsi une flexion de la portion fléchissable.

Selon un mode de réalisation, les transducteurs à ultrasons de la sonde ultrasonore sont répartis selon plusieurs couronnes concentriques autour de la lumière axiale, chaque couronne comprenant chacune plusieurs transducteurs répartis en secteurs radiaux le long de ladite couronne, par exemple le nombre de couronnes est supérieur à 3 et le nombre de transducteurs par couronne est supérieur à 30.

Selon un mode de réalisation, les transducteurs à ultrasons sont des transducteurs piézoélectriques et sont formés par plusieurs couches annulaires s'étendant l'une sur l'autre autour de la lumière axiale, lesdites couches annulaires comprenant :
- une couche piézoélectrique métallisée sur chacune de ses faces interne et externe, et divisée en plusieurs secteurs piézoélectriques, par exemple des secteurs annulaires et radiaux ;
- une couche d'adaptation d'impédance sur la couche piézoélectrique et divisée en plusieurs secteurs d'adaptation d'impédance, chaque secteur d'adaptation d'impédance étant positionné en vis-à-vis d'un secteur piézoélectrique ; et
- une couche d'atténuation acoustique, sous la couche piézoélectrique.

Selon un mode de réalisation, la gaine d'introduction médicale comprend en outre une structure d'interconnexion incluant les bandes de connexion, la structure d'interconnexion comprenant en outre une portion annulaire disposée entre la couche d'atténuation acoustique et la couche piézoélectrique, ladite portion annulaire comprenant des pistes métalliques reliées aux transducteurs à ultrasons et se prolongeant dans les bandes de connexion.

Selon un mode de réalisation, la gaine d'introduction médicale comprend en outre un embout annulaire à l'extrémité distale, ledit embout étant relié au coupleur d'orientation, par exemple à un maillon distal dudit coupleur d'orientation, les transducteurs à ultrasons étant disposés dans une gorge circonférentielle dudit embout tout autour de la lumière axiale.

Selon un mode de réalisation, la gaine d'introduction médicale comprend en outre une enveloppe externe autour des bandes de connexion, du fourreau et de la structure élastique, l'enveloppe externe étant par exemple en un matériau biocompatible.

Un mode de réalisation prévoit un dispositif d'exploration et d'intervention intracorporelles comprenant une gaine d'introduction médicale comme décrit précédemment.

Selon un mode de réalisation, le dispositif comprend en outre un cathéter configuré pour être introduit dans la lumière axiale de la gaine d'introduction médicale.

Selon un mode de réalisation, le dispositif comprend en outre, à l'extrémité proximale, une poignée de contrôle adaptée à contrôler une flexion de la portion fléchissable.

Selon un mode de réalisation, la gaine d'introduction médicale est un trocart.

Un mode de réalisation prévoit une méthode d'utilisation de la gaine d'introduction.

Selon un mode de réalisation, la méthode d'utilisation comprend une utilisation de la gaine d'introduction pour un traitement d'une pathologie cardiaque, par exemple pour implanter un stimulateur cardiaque, pour réaliser une ablation par radiofréquence, pour remplacer ou implanter une valve cardiaque.

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :
la figure 1 est une vue d'ensemble d'un dispositif d'exploration et d'intervention intracorporelles selon un mode de réalisation ;
la figure 2A, la figure 2B et la figure 2C sont des vues en coupe longitudinale représentant partiellement un exemple de gaine d'introduction médicale selon un mode de réalisation ;
la figure 3A et la figure 3B sont des vues en trois dimensions de la gaine d'introduction médicale des figures 2A à 2C ;
la figure 3C et la figure 3D sont des vues représentant la structure d'interconnexion de la gaine d'introduction médicale des figures 3A et 3B ; et
la figure 4 est une vue schématique représentant un exemple de sonde d'imagerie ultrasonore d'une gaine d'introduction médicale selon un mode de réalisation.

### Description des modes de réalisation

De mêmes éléments ont été désignés par de mêmes références dans les différentes figures. En particulier, les éléments structurels et/ou fonctionnels communs aux différents modes de réalisation peuvent présenter les mêmes références et peuvent disposer de propriétés structurelles, dimensionnelles et matérielles identiques.

Par souci de clarté, seuls les étapes et éléments utiles à la compréhension des modes de réalisation décrits ont été représentés et sont détaillés. En particulier, les transducteurs à ultrasons des sondes ultrasonores décrites n'ont pas été détaillés, les modes de réalisation décrits étant compatibles avec toutes ou la plupart des structures connues de transducteurs à ultrasons.

Sauf précision contraire, lorsque l'on fait référence à deux éléments connectés entre eux, cela signifie directement connectés sans éléments intermédiaires autres que des conducteurs, et lorsque l'on fait référence à deux éléments reliés (en anglais "coupled") entre eux, cela signifie que ces deux éléments peuvent être connectés ou être reliés par l'intermédiaire d'un ou plusieurs autres éléments.

Sauf précision contraire, lorsque l'on fait référence à deux éléments montés, ou positionnés, l'un sur l'autre, cela ne signifie pas nécessairement que ces deux éléments sont montés, ou positionnés, directement l'un sur l'autre, un ou plusieurs autres éléments pouvant être positionnés entre ces deux éléments.

Dans la description qui suit, lorsque l'on fait référence à des qualificatifs de position absolue, tels que les termes "avant", "arrière", "haut", "bas", "gauche", "droite", etc., ou relative, tels que les termes "dessus", "dessous", "supérieur", "inférieur", etc., ou à des qualificatifs d'orientation, tels que les termes "horizontal", "vertical", etc., il est fait référence sauf précision contraire à l'orientation des figures.

Sauf précision contraire, les expressions "environ", "approximativement", "sensiblement", et "de l'ordre de" signifient à 10 % ou à 10° près, de préférence à 5 % ou à 5° près.

Dans la description qui suit, sauf précision contraire, lorsque l'on fait référence à un transducteur, il est fait référence à un transducteur à ultrasons, lorsque l'on fait référence à une sonde, il est fait référence à une sonde d'imagerie ultrasonore, et lorsque l'on fait référence à une gaine ou à une gaine d'introduction, il est fait référence à une gaine d'introduction médicale. Une gaine d'introduction médicale peut, par exemple, être un trocart ou une canule, ou tout autre instrument médical permettant de former une voie dans une région anatomique.

Dans la description qui suit, lorsque l'on fait référence à un cathéter, il est fait référence, selon une définition large, à un dispositif en forme de tige fine, creuse ou pleine, comprenant généralement au moins une portion fléchissable, et destiné à être introduit dans une région d'un corps humain ou animal (par exemple une cavité, une lumière ou lumen, ou un conduit).

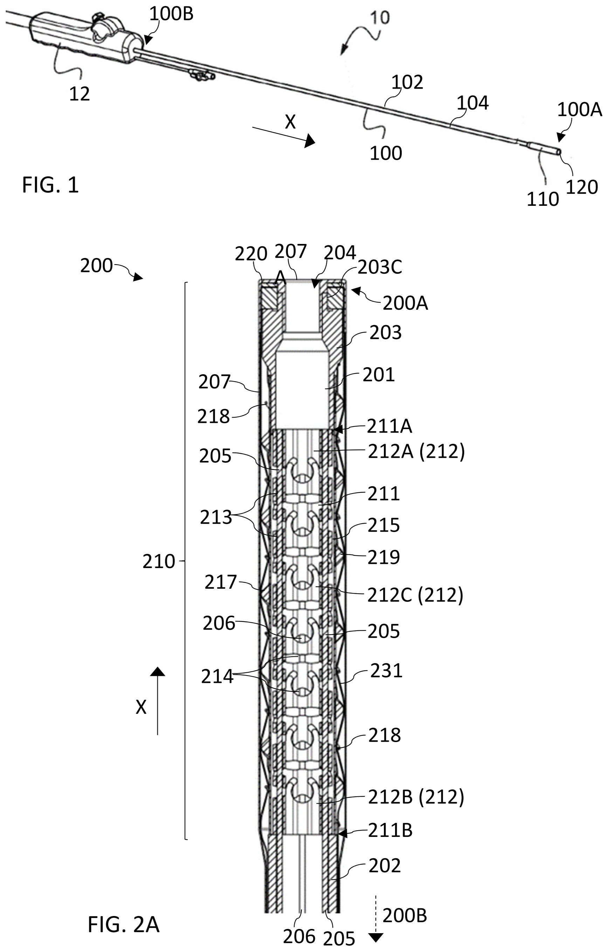

La figure 1 est une vue d'ensemble d'un dispositif 10 d'exploration et d'intervention intracorporelles selon un mode de réalisation.

Le dispositif 10 comprend une gaine d'introduction médicale 100, et une poignée de contrôle 12 reliée à l'extrémité proximale 100B de la gaine 100. La gaine 100 est destinée à destinée à être introduite dans une région anatomique humaine ou animale, par exemple pour explorer et/ou intervenir dans cette région anatomique.

La gaine 100 présente sur sa longueur une forme de tige tubulaire 102, définissant une lumière axiale 104 interne à la tige tubulaire 102. La lumière axiale 104 est adaptée à recevoir un cathéter, ou tout autre appareil médical allongé (non représenté). Par exemple, la lumière axiale 104 interne est dimensionnée pour recevoir de manière coulissante et/ou rotative un cathéter. La longueur du cathéter peut être sensiblement égale à la longueur de la gaine 100, et de la tige tubulaire 102.

La gaine 100 comprend une portion fléchissable 110, ou portion orientable, c'est-à-dire une portion qui peut être courbée pour s'adapter à l'anatomie de la région dans laquelle elle est introduite, par exemple jusqu'à un angle compris entre 90° et 180° par rapport à la direction axiale X. La portion fléchissable 110 s'étend sur une portion de la gaine 100 jusqu'à l'extrémité distale 100A de la gaine 100. La portion fléchissable 110 présente, par exemple, une longueur de quelques centimètres (cm), par exemple environ 4 cm.

La poignée de contrôle 12 est adaptée à contrôler l'avancement et le positionnement de la gaine 100, ainsi que la flexion, ou courbure, de la portion fléchissable 110 de la gaine 100.

La gaine 100 comprend, à son extrémité distale 100A, qui est aussi l'extrémité distale de la portion fléchissable 110, une sonde d'imagerie ultrasonore 120. Par exemple, la sonde 120 s'étend tout autour de la lumière axiale 104 au niveau de l'extrémité distale 100A.

Dans l'ensemble de la description, le terme "proximal" (ou "arrière") est considéré par rapport au dispositif dans son ensemble, c'est-à-dire en direction de la poignée de contrôle, et le terme "distal" (ou "avant") se réfère à une direction opposée, en direction de la région d'exploration et/ou d'intervention (région cible). L'extrémité distale de la gaine d'introduction médicale correspond à l'extrémité par laquelle cette gaine est introduite dans la région cible, et l'extrémité proximale correspond à l'extrémité opposée à l'extrémité distale.

Dans l'ensemble de la description, le terme "axial" est utilisé en référence à l'axe, dans la direction X, du dispositif 10, c'est-à-dire la plus grande dimension (longueur) de celui-ci, correspondant également à la plus grande dimension (longueur) de la gaine 100, et une direction "radiale" est une direction située dans un plan perpendiculaire à la direction axiale X. Par longitudinal, on fait référence à une direction parallèle à la direction axiale X, et par transversal, on fait référence à un plan ou une direction perpendiculaire à la direction axiale X. Une coupe longitudinale est une coupe réalisée dans un plan incluant la direction X, tandis qu'une coupe transversale est une coupe réalisée dans un plan perpendiculaire à la direction X incluant la direction radiale.

La sonde 120 est orientée en face avant ("forward looking" en anglais), c'est-à-dire orientée depuis l'extrémité distale 100A de la gaine 100 dans la direction d'avancement de la gaine 100. En d'autres termes, la gaine 100 munie de la sonde 120 à son extrémité distale 100A est capable de transmettre et de recevoir des signaux ultrasonores dans une direction généralement orientée vers l'avant. La sonde 120 permet de visualiser les régions anatomiques qui sont situées face à l'extrémité distale 100A de la gaine 100. La sonde 120 permet notamment de visualiser la région anatomique cible pendant l'intervention, voire avant et/ou après l'intervention, et ce, sans avoir à introduire un autre cathéter muni d'une sonde d'imagerie.

Par exemple, la sonde 120 peut fournir des images ultrasonores des régions anatomiques en temps réel dans le sens d'avancement de la gaine 100 au travers du corps du patient. Par exemple, les images ultrasonores générées à partir de la sonde 120 peuvent être utilisées pour guider la gaine 100 vers la région cible, confirmer le contact tissulaire d'un cathéter dans la région cible, déterminer l'orientation de la gaine 100 et/ou du cathéter dans le corps du patient, surveiller la progression d'une lésion en cours de formation dans le tissu, voire surveiller les structures anatomiques adjacentes, par exemple afin d'éviter des effets collatéraux indésirables sur ces structures, surveiller la progression et l'efficacité du traitement ...

Avantageusement, la gaine 100 munie de la sonde 120 peut être utilisée pour déployer un cathéter dans la région anatomique cible. Avantageusement, la gaine 100 munie de la sonde 120 peut réduire considérablement, voire supprimer, l'utilisation de la radioscopie comme moyen de visualisation de la gaine 100, et du cathéter, pendant une intervention. En d'autres termes, le guidage ultrasonore apporté par la gaine 100 permet de limiter, voire de s'affranchir, du guidage radioscopique pour l'introduction de la gaine jusqu'à une région cible.

Dans une forme particulière, non limitative, de mise en œuvre des modes de réalisation, la gaine 100 peut être utilisée, en association avec un cathéter positionné dans la lumière axiale 104, pour implanter un stimulateur cardiaque ("pacemarker" en anglais), pour réaliser une ablation de tissus par radiofréquence (RF ablation) ou par cryoablation, ou pour remplacer ou implanter une valve cardiaque, par exemple réaliser une implantation de valve aortique transcathéter (TAVI, de l'anglais "Transcatheter Aortic Valve Implantation"), ou un remplacement de valve aortique transcathéter (TAVR, de l'anglais " Transcatheter Aortic Valve Replacement"). Les modes de réalisation ne sont cependant pas limités à ces utilisations, ou à une utilisation clinique spécifique.

La sonde 120 comprend par exemple un réseau de transducteurs à ultrasons, de préférence sous forme d'une matrice de transducteurs à ultrasons.

Un transducteur à ultrasons est un transducteur adapté à convertir un signal électrique en onde ultrasonore, et inversement à convertir une onde ultrasonore en signal électrique. Selon le type du transducteur, le signal électrique peut correspondre à une tension, un courant, ou une charge électrique.

Le réseau de transducteurs peut comprendre tout type de transducteurs à ultrasons, voire plusieurs types de transducteurs à ultrasons.

Les transducteurs à ultrasons peuvent être constitués d'une couche de matériau piézoélectrique monocristallin ou polycristallin, par exemple du PZT (Titanate de Plomb-Zircone), ou une structure composite comprenant au moins une couche de matériau piézoélectrique, par exemple une couche de PZT incluant des rainures remplies de polymère.

Les transducteurs à ultrasons peuvent être des microsystèmes électromécaniques, ou MEMS, de l'anglais Micro-Electro-Mechanical System, mettant en œuvre des technologies de production de la micro-électronique. Un transducteur du type MEMS comprend généralement un ou plusieurs éléments acoustiques comprenant chacun une (ou plusieurs) membrane(s) déformable(s) suspendue(s) au-dessus d'une cavité et reliée(s) par une électrode commune. Selon un mode de réalisation, chaque membrane déformable est déplacée ou déformée par effet capacitif à l'aide d'une électrode fixée à cette membrane et d'une électrode séparée par la cavité. Ce type de transducteur à ultrasons est connu sous l'acronyme CMUT, de l'anglais Capacitive Micro-machined Ultrasonic Transducer, c'est-à-dire un transducteur capacitif micro-usiné à ultrasons, ou transducteur capacitif à membrane. Selon un autre mode de réalisation, chaque membrane déformable est déplacée ou déformée par effet piézoélectrique à l'aide d'une couche de matériau piézoélectrique munie de deux électrodes attachées à la membrane. Ce type de transducteur à ultrasons est connu sous l'acronyme PMUT, de l'anglais Piezoelectric Micro-machined Ultrasonic Transducer c'est-à-dire un transducteur piézoélectrique micro-usiné à ultrasons, ou transducteur piézoélectrique à membrane.

Il se pose la problématique de réaliser une liaison, en particulier électrique, voire optique, entre les transducteurs à ultrasons de la sonde d'imagerie ultrasonore 120 à l'extrémité distale 100A de la gaine et l'extrémité proximale 100B de la gaine 100 où se trouve la poignée de contrôle 12.

Les transducteurs à ultrasons sont généralement reliés à une structure d'interconnexion au niveau de la sonde 120, et en particulier à des pistes conductrices, généralement métalliques, de la structure d'interconnexion, la structure d'interconnexion se prolongeant par des bandes de connexion (conductrices), par exemple des câbles, des nappes, des lames, ou des limandes, de connexion, reliées aux pistes conductrices de la structure d'interconnexion. Les bandes de connexion s'étendent dans la direction axiale X vers l'extrémité proximale 100B de la gaine 100.

Une difficulté majeure pour réaliser la liaison est liée à la flexion de la gaine 100, du moins de la portion fléchissable 110 de la gaine 100 qui subit des déformations importantes et opposées entre l'intérieur et l'extérieur de la flexion. Idéalement, les bandes de connexion devraient passer par le centre de la gaine 100, c'est-à-dire dans la lumière axiale 104 interne de la gaine 100, de préférence au plus près de l'axe de la gaine 100, de façon à minimiser les contraintes/déformations liées à l'éloignement par rapport à la ligne neutre lors de la flexion de la gaine 100. Cependant, comme la lumière axiale 104 est destinée au passage d'un cathéter ou tout autre appareil médical allongé, les bandes de connexion ne peuvent pas passer dans la lumière axiale 104, et elles passent en périphérie de la gaine 100. Ainsi, les bandes de connexion s'éloignent de l'axe de la gaine 100, et de la ligne neutre, d'autant plus que le diamètre de la gaine 100 est important.

Lorsque la gaine 100 est fléchie, une moitié hémicylindrique est soumise à un allongement (extension) du côté opposé à la courbure, pendant que l'autre moitié est soumise, avec une même amplitude, à une contraction (compression). L'allongement et la contraction générés par la courbure sont d'autant plus importants que l'on s'éloigne de l'axe de la gaine 100 et que l'on s'approche du plan de courbure. Les bandes de connexion passant en périphérie de la gaine 100, sur le plan de courbure, subissent également l'allongement et la contraction générés par la courbure. Une solution existante pour éviter que les bandes de connexion ne subissent ces contraintes, ou du moins pour les limiter, est de les enrouler autour de la gaine 100, par exemple de manière sensiblement hélicoïdale, afin de répartir de façon homogène les zones soumises à l'allongement et à la contraction. Cependant, outre la difficulté technique potentielle à réaliser ce type de solution dans les dimensions contraignantes des gaines d'introduction, allant de quelques millimètres à quelques dizaines de millimètres de diamètre extérieur, l'enroulement conduit inévitablement à allonger les bandes de connexion, ce qui conduit à augmenter d'autant les pertes électriques.

En outre, dans le cas de réseaux de transducteurs à haute densité de transducteurs, l'interconnexion des transducteurs est généralement à haute densité, dans des dimensions petites et contraintes, ce qui peut amplifier les pertes électriques et généralement les coûts.

Les figures 2A à 3D qui suivent illustrent une solution répondant à la problématique de réaliser une liaison entre les transducteurs à ultrasons de la sonde d'imagerie ultrasonore 120 et la poignée de contrôle 12, qui permette de gérer les problèmes d'allongement et de contraction générés par la courbure de la gaine 100, tout en limitant la longueur des bandes de connexion, réduisant ainsi les pertes électriques associées.

Il est en outre recherché une solution permettant de répondre à la problématique d'allongement et de contraction générés par la courbure de la gaine, même avec un réseau de transducteurs à haute densité de transducteurs et une haute densité d'interconnexions permettant l'adressage individuel ou du type RCA de ces transducteurs.

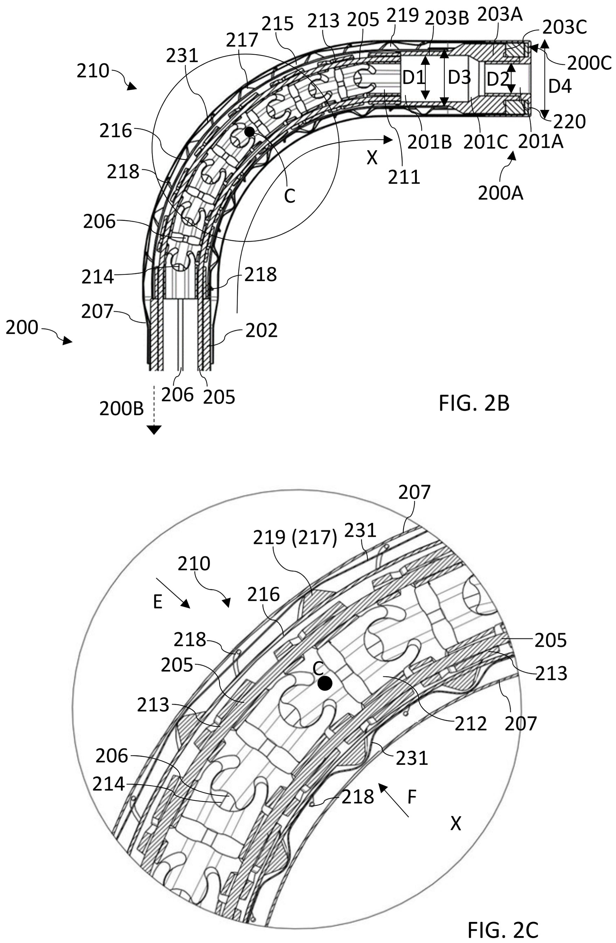

La figure 2A, la figure 2B et la figure 2C sont des vues en coupe longitudinale représentant partiellement un exemple de gaine d'introduction médicale 200 selon un mode de réalisation. Les figures 2A à 2C représentent plus particulièrement la portion fléchissable 210 de la gaine d'introduction 200, ou portion distale 210. La figure 2A est une vue de la portion distale 210 dans une configuration droite. La figure 2B est une vue de la portion distale 210 dans une configuration courbée à environ 90°. La figure 2C est une vue de détail de la portion distale 210 prise dans le cercle représenté dans la figure 2B.

Lorsque la gaine est courbée, on peut parler de gaine béquillée dans le domaine technique de la présente description.

Dans la figure 2A, la direction axiale X est droite, tandis que dans les figures 2B et 2C, la direction axiale X est fléchie.

La gaine d'introduction 200 représentée dans les figures 2A à 2C peut correspondre à la gaine 100 de la figure 1, la portion distale 210 correspondant alors à la portion fléchissable 110 de la figure 1.

La gaine 200 est creuse, avec une lumière axiale 204 interne qui s'étend le long de la direction axiale X, entre les extrémités distale 200A et proximale 200B de la gaine 200.

La portion distale 210 de la gaine d'introduction médicale 200 comprend un coupleur d'orientation 211, qui est adapté à conférer une courbure à la portion distale 210.

Le coupleur d'orientation 211 comprend une pluralité de maillons 212, deux maillons adjacents coopérant l'un avec l'autre de manière à pouvoir pivoter autour d'au moins un axe de rotation. Par exemple, les maillons 212 forment une articulation.

La pluralité de maillons 212 comprend notamment :
- un maillon distal 212A à l'extrémité distale 211A du coupleur 211 ;
- un maillon proximal 212B à l'extrémité proximale 211B du coupleur 211 ; et
- des maillons intermédiaires 212C entre le maillon distal 212A et le maillon proximal 212B.

Les maillons 212 sont par exemple en un matériau de nature à constituer des paliers lisses entre les maillons, par exemple :
- un métal : par exemple un acier, l'aluminium, le tungstène, le titane ... ;
- un matériau polymère rigide : par exemple un polycarbonate (PC), un polyméthacrylate de méthyle (PMMA) ... ;
- en matériau céramique : par exemple l'alumine (Al₂O₃), un oxide de zirconium (ZrO₂), un carbure de silicium (SiC)...

Les maillons 212 sont par exemple des rotules. La personne du métier pourra déterminer d'autres types de maillons aptes à pivoter l'un par rapport à l'autre autour d'au moins un axe de rotation, de manière à conférer une courbure à la portion fléchissable 210 de la gaine 200.

Le maillon distal 212A est relié à une bague distale 201. Le maillon proximal 212B est relié à une bague proximale 202, par exemple est emmanché dans la bague proximale 202. Les bagues 201 et 202 peuvent former des raidisseurs. La bague distale 201 peut être désignée "tête de béquillage".

La bague distale 201 est elle-même emmanchée dans un embout 203 qui est emboîté avec le maillon distal 212A, de sorte à former un ensemble bague distale/embout/maillon distal solidaire. L'embout 203 est par exemple un embout annulaire.

Dans l'exemple représenté, la bague distale 201 présente un diamètre qui rétrécit vers l'extrémité distale 200A de la gaine 200. Ainsi, la bague distale 201 comprend une portion proximale 201B cylindrique d'un diamètre D1, une portion distale 201A cylindrique d'un diamètre D2 inférieur au diamètre D1, et une portion tronconique 201C reliant les portions 201A et 201B.

Dans l'exemple représenté, l'embout 203 présente un diamètre qui augmente vers l'extrémité distale 200A de la gaine 200. Ainsi, l'embout 203 comprend une portion proximale 203B cylindrique d'un diamètre D3 (qui s'emboite avec le maillon distal 212A), une portion distale 203A cylindrique d'un diamètre D4 supérieur au diamètre D3, et une portion tronconique reliant les portions 203A et 203B.

Les bagues 201, 202 et l'embout 203 sont creux, par exemple annulaires, et les maillons 212 sont en forme d'anneaux, ou du moins en une forme permettant de délimiter une portion centrale creuse, de manière à ce que la gaine 200 conserve une lumière axiale 204, y compris dans la portion distale 210.

Les maillons 212 sont maintenus les uns contre les autres par des câbles 205, respectivement 206, ou câbles de tringlerie, par exemple des câbles métalliques, qui passent dans des gorges 213, respectivement 214, formées sur des périphéries des maillons 212.

Deux câbles 205 constituent un premier couple de câbles parallèles entre eux dans le plan des figures 2A à 2C, tandis que deux autres câbles 206 constituent un deuxième couple de câbles parallèles entre eux dans un plan perpendiculaire au plan des figures 2A à 2C. Par exemple, les câbles 205 permettent de courber la portion distale 210 dans une direction perpendiculaire à la direction X et un plan parallèle au plan des figures 2A à 2C, et les câbles 206 permettent de courber la portion distale 210 dans une direction perpendiculaire à la direction X et perpendiculaire au plan des figures 2A à 2C.

Par exemple, les câbles 205 et 206 ont chacun une extrémité distale retenue dans le maillon distal 212A, dans la bague 201, ou dans l'embout 203.

Les câbles 205 et 206 se prolongent de préférence jusqu'à l'extrémité proximale 200B de la gaine 200, par exemple jusqu'à la poignée de contrôle 12 visible dans la figure 1. Les câbles 205 et 206 peuvent ainsi être contrôlés de manière à contrôler les pivotements des maillons 212, et ainsi la flexion de la portion distale 210.

La portion distale 210 de la gaine d'introduction médicale 200 comprend en outre un fourreau 215, qui est disposé autour du coupleur d'orientation 211, c'est-à-dire autour des maillons 212.

Le fourreau 215 peut être emboîté, en étant autour du coupleur d'orientation 211, dans la bague proximale 202. Le fourreau 215 peut se prolonger autour de l'embout 203, par exemple autour de la portion proximale 203B de l'embout 203.

Le fourreau 215 est en un matériau permettant une flexibilité, de sorte que le fourreau 215 peut suivre la courbure de la portion distale 210. Par exemple, le fourreau 215 peut être en un matériau élastomère, par exemple un élastomère thermoplastique (TPE), par exemple en un polyéther bloc amide (PEBA), par exemple en silicone. Le fourreau 215 peut être en un matériau biocompatible, mais ce n'est pas obligatoire, le fourreau n'étant pas en contact avec le milieu extérieur de la gaine 200, c'est-à-dire avec le milieu qui entoure la gaine 200 ou qui est à l'intérieur de la gaine 200, dans la lumière 204.

Le fourreau 215 comprend une portion tubulaire 216 sensiblement cylindrique et une portion périphérique 217 faisant saillie sur la paroi externe de la portion tubulaire 216. La portion périphérique 217 comprend une pluralité de tours en saillie 219, ou protrusions. Les protrusions 219 font saillie dans la direction radiale vers l'extérieur, c'est-à-dire en s'éloignant de l'axe, de sorte que la portion tubulaire 216 est comprise entre les protrusions 219 et les maillons 212.

Dans l'exemple représenté, la portion périphérique 217 présente une forme hélicoïdale, protubérante, qui s'enroule autour et le long de la portion tubulaire 216 dans la direction axiale X. Les protrusions 219 correspondent aux tours de l'hélice, et sont ainsi reliées entre elles. Le pas de l'hélice peut être régulier, ou être irrégulier.

En variante, la portion périphérique peut comprendre plusieurs anneaux en saillie disjoints entre eux et enroulés autour de la portion tubulaire 216. Les anneaux en saillie sont répartis, par exemple régulièrement répartis, sur plusieurs circonférences de la portion tubulaire 216 le long de la direction axiale X, les anneaux en saillie formant les protrusions. Dans ce cas, les tours en saillie formant les protrusions sont disjoints entre eux.

La gaine 200 comprend, à son extrémité distale 200A, qui est aussi l'extrémité distale de la portion distale 210, une sonde d'imagerie ultrasonore 220 dite forward looking, c'est-à-dire orientée depuis l'extrémité distale 200A de la gaine 200 dans la direction d'avancement de la gaine 200, comme défini plus avant. La sonde 220 s'étend tout autour de la lumière axiale 204 sur la face transversale 200C de la gaine 200 qui est à l'extrémité distale 200A. La face transversale 200C est une face perpendiculaire à la direction axiale X. La face transversale 200C est dans cet exemple sous la forme d'une couronne, et est tout autour de la lumière axiale 204.

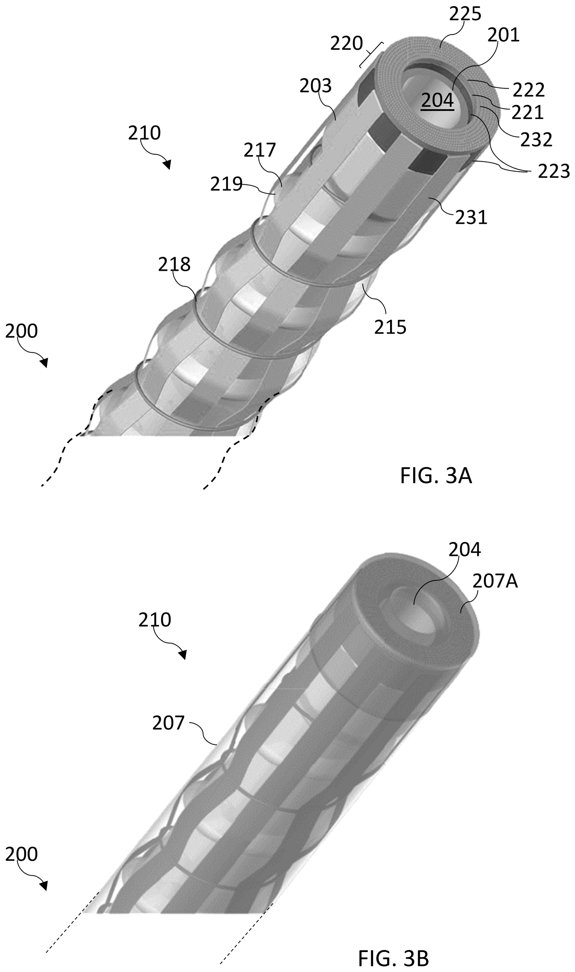

La sonde 220 comprend un réseau de plusieurs transducteurs à ultrasons 225 (repérés dans la figure 3A) qui sont positionnés au moins en partie dans une gorge circonférentielle 203C formée dans l'embout 203 tout autour de la lumière axiale 204.

Le réseau de transducteurs est de préférence une matrice de transducteurs.

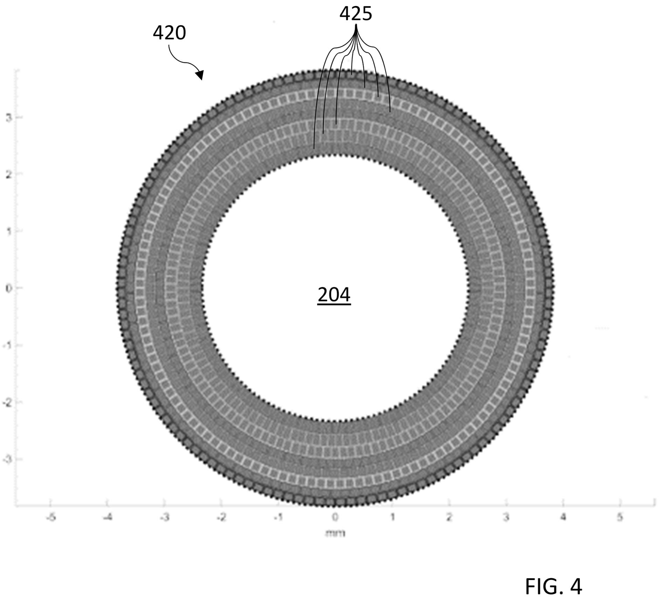

Le réseau de transducteurs peut comprendre tout nombre de transducteurs à ultrasons, par exemple entre 128 et 1024, par exemple comprendre plusieurs couronnes concentriques comprenant chacune plusieurs transducteurs à ultrasons. Les transducteurs à ultrasons d'une même couronne peuvent être répartis en secteurs radiaux de la couronne, comme illustré dans l'exemple de la figure 4 décrite plus après.

Par exemple, le nombre de couronnes est supérieur à 3 et le nombre de transducteurs (secteurs radiaux) par couronne est supérieur à 30.

Les transducteurs sont généralement reliés à une structure d'interconnexion 230 (décrite plus après en lien avec les figures 3A à 3D) au niveau de la sonde 220, et en particulier reliés à des pistes conductrices de la structure d'interconnexion. La structure d'interconnexion se prolonge par des bandes de connexion (conductrices) 231, par exemple des câbles, des nappes, des lames, ou des limandes de connexion, reliées aux pistes conductrices de la structure d'interconnexion. Dans la suite de la description, les bandes de connexion 231 sont désignées par le terme de limandes 231.

Les limandes 231 sont par exemple réalisées chacune en un circuit imprimé flexible. Un circuit imprimé flexible est constitué de pistes conductrices, par exemple en cuivre, disposées sur ou à l'intérieur d'un substrat isolant flexible en un matériau diélectrique, généralement un polymère, par exemple du polyimide.

Les limandes 231 s'étendent dans la direction axiale X depuis la sonde 220 en direction de l'extrémité proximale 200B de la gaine 200. Par exemple, les limandes 231 sont sensiblement droites dans la configuration de la figure 2A (gaine non courbée).

Les limandes 231 passent en périphérie de la gaine 200, en particulier autour, et le long, du fourreau 215. Par exemple, les limandes 231 sont régulièrement réparties autour du fourreau 215.

Les limandes 231 peuvent se prolonger jusqu'à l'extrémité proximale 200B de la gaine 200. En variante, les limandes 231 peuvent s'interrompre avant l'extrémité proximale 200B de la gaine 200, et par exemple être reliées à une nappe flexible conductrice qui s'étend jusqu'à l'extrémité proximale 200B, voire au-delà.

La portion distale 210 de la gaine d'introduction médicale 200 comprend en outre une structure élastique 218 enroulée autour du fourreau 215 et des limandes 231. La structure élastique 218 chemine selon plusieurs circonférences entre les protrusions 219.

La structure élastique 218 est adaptée, lors de la courbure de la portion distale 210, pour absorber l'allongement (extension) d'un côté E (côté opposé à la courbure, ou côté du grand rayon de courbure) de la gaine 200, et la contraction (compression) de l'autre côté F (côté courbure, ou côté du petit rayon de courbure) de la gaine 200, avec les limandes 231 qui restent étendues dans la direction axiale X, c'est-à-dire sans avoir à enrouler les limandes 231 autour de la gaine 200, permettant ainsi de limiter les longueurs des limandes, et limitant d'autant les pertes électriques.

Comme on peut le voir dans la figure 2C :
- du côté E opposé à la courbure, les limandes 231 sont sensiblement tendues entre les protrusions 219 et la structure élastique 218 est en extension de ce côté E ; et
- du côté F de la courbure, la structure élastique 218 en compression vient plaquer les limandes 231 contre le fourreau 215, et notamment contre les protrusions 219 et contre la portion tubulaire 216 entre les protrusions 219.

Ainsi, les protrusions 219, par le profil qu'elles forment avec la portion tubulaire 216, combinées avec la structure élastique 218, permettent que les limandes 231 aient toutes sensiblement la même longueur. La personne du métier pourra déterminer l'épaisseur, et éventuellement la largeur dans la direction X, des protrusions 219 de manière à obtenir cet effet.

La structure élastique 218 est en un matériau suffisamment souple pour être enroulé autour des limandes 231, par exemple en caoutchouc, en un élastomère, en un polymère.

La section de la structure élastique 218 est par exemple circulaire, ovale, rectangulaire, ou polygonale. La structure élastique 218 peut également être constituée d'un fil rigide structuré sous forme de ressort.

On notera que la structure élastique 218 reste peu déformée lorsque l'on passe d'une configuration droite (Figure 2A) à une configuration courbée (Figure 2B). En effet, la structure élastique 218 s'éloigne du centre de courbure sans être sensiblement déformée. L'élasticité de la structure 218, tout comme sa section, sont de préférence à adapter par la personne du métier pour absorber les déformations des limandes 231.

Dans l'exemple représenté, la structure élastique 218, de même que la portion périphérique 217, est sous une forme d'hélice, à plusieurs tours, cheminant autour et le long du fourreau 215 dans la direction axiale X. Les tours de la structure élastique 218 cheminent entre les protrusions 219 de la portion périphérique 217.

En variante, la structure élastique, et dans ce cas la portion périphérique, peut être sous la forme de plusieurs anneaux disjoints entre eux. Dans ce cas, les tours de la structure élastique sont disjoints entre eux. Les anneaux de la structure élastique sont positionnés entre les anneaux en saillie de la portion périphérique.

Les tours de la structure élastique 218 et les tours de la portion périphérique 217, qu'ils soient joints ou disjoints sous la forme d'anneaux, peuvent être répartis à intervalles réguliers dans la direction X.

En variante, les tours de la structure élastique 218 et les tours de la portion périphérique 217, qu'ils soient joints ou disjoints sous la forme d'anneaux, peuvent être répartis de manière irrégulière, par exemple avec une densité accrue autour du centre C de la portion distale 210, où la courbure atteint son maximum.

Le nombre de tours de la structure élastique 218 est par exemple compris entre 5 et 150. Plus généralement, la personne du métier saura déterminer le nombre de tours en fonction de l'angle de flexion de la portion distale 210, du diamètre extérieur de la gaine 200, du rayon de courbure, de la capacité d'absorption des protrusions 219. La personne du métier pourra choisir de répartir la structure élastique 218 sur sensiblement toute la longueur de la gaine 200 et non seulement autour de la portion fléchissable 210, ce qui peut augmenter le nombre de tours.

Une enveloppe externe 207 sous la forme d'une gaine souple enveloppe l'ensemble des éléments décrits ci-dessus, et notamment les limandes 231, la structure élastique 218, le fourreau 215, les bagues 201, 202, l'embout 203, et les câbles 205, 206.

Cette enveloppe externe 207 peut comprendre une extrémité distale 207A positionnée sur la sonde 220, et, dans ce cas, l'enveloppe externe 207 est de préférence transparente aux ondes ultrasonores, par exemple est en silicone.

L'enveloppe externe 207 est de préférence biocompatible, par exemple en silicone, par exemple en PEBA, ou bien revêtue d'un matériau biocompatible, par exemple revêtue de parylène.

Une enveloppe interne (non visible sur les figures 2A à 2C) peut être prévue autour de la portion distale 201A de la bague distale 201, la portion distale 203A de l'embout 203 étant alors autour de cette enveloppe interne. L'enveloppe interne est par exemple en silicone.

A titre d'illustration, non limitative, le plus grand diamètre de la lumière axiale 204, c'est-à-dire le diamètre interne de la portion distale 210 de la gaine 200, est compris entre 2,5 et 25 mm, et le diamètre hors tout de la portion distale 210 de la gaine 200, c'est à dire le diamètre externe de l'enveloppe externe 207, est compris entre 7 et 30 mm.

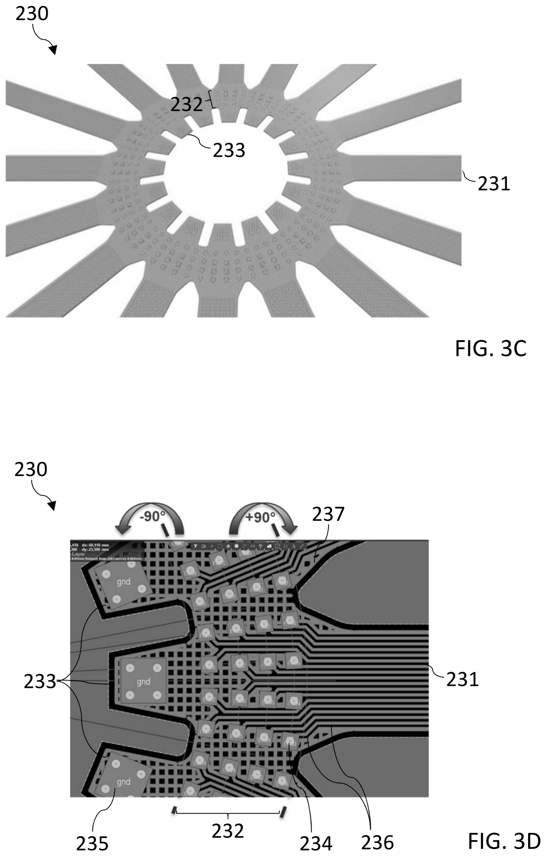

La figure 3A et la figure 3B sont des vues en trois dimensions de la gaine d'introduction médicale 200 des figures 2A à 2C. La figure 3C et la figure 3D sont des vues représentant la structure d'interconnexion 230 de la gaine d'introduction médicale des figures 3A et 3B. La figure 3A représente en trois dimensions la portion fléchissable 210 sans l'enveloppe externe, tandis que la figure 3B représente en trois dimensions la portion fléchissable 210 avec l'enveloppe externe 207. La figure 3C représente en trois dimensions un détail de la structure d'interconnexion 230 déployée perpendiculairement à la direction X. La figure 3D représente un autre détail de la structure d'interconnexion 230 dans une direction parallèle à la direction X.

La gaine d'introduction représentée dans les figures 3A et 3B correspond à la gaine 200 des figures 2A à 2C, la portion fléchissable correspondant à la portion distale 210 des figures 2A à 2C.

Dans l'exemple de la figure 3A, les transducteurs à ultrasons 225 de la sonde d'imagerie ultrasonore 220 sont des transducteurs à base de matériau piézoélectrique et sont formés par plusieurs couches annulaires s'étendant l'une sur l'autre autour de la lumière axiale 204 :
- une couche en matériau piézoélectrique 221, ou couche piézoélectrique 221, découpée sur toute son épaisseur pour former plusieurs secteurs de la couche piézoélectrique, ou secteurs piézoélectriques : la couche piézoélectrique peut être métallisée sur chacune de ses faces interne et externe pour former une couche métallique externe et une couche métallique interne, la découpe de la couche piézoélectrique incluant la découpe des couches métalliques externe et interne, chaque secteur piézoélectrique comprenant ainsi une électrode externe, correspondant à un secteur de la couche métallique externe découpée, et une électrode interne correspondant à un secteur de la couche métallique interne découpée ; et
- une couche d'adaptation d'impédance 222 sur la couche piézoélectrique : la couche d'adaptation d'impédance est découpée, généralement en même temps que la couche piézoélectrique, pour former plusieurs secteurs de la couche d'adaptation d'impédance, ou secteurs d'adaptation d'impédance, chaque secteur d'adaptation d'impédance étant positionné en vis-à-vis d'un secteur piézoélectrique, et par exemple en contact avec ce secteur piézoélectrique, formant un empilement.

Au lieu d'une seule couche piézoélectrique, il peut s'agir d'un empilement de couches piézoélectriques.

Un empilement d'un secteur d'adaptation d'impédance sur un secteur piézoélectrique permet de former tout ou partie d'un transducteur à ultrasons 225. Les empilements de secteurs d'adaptation d'impédance et piézoélectrique sont généralement séparés entre eux par des fentes, ou kerfs.

Les secteurs peuvent être annulaires et radiaux, de sorte que le réseau de transducteurs comprend des couronnes comprenant chacune plusieurs transducteurs 225 répartis le long de la circonférence de la couronne.

Une couche annulaire en un matériau d'atténuation acoustique 223, ou couche de "backing", est positionnée sous la couche piézoélectrique 221. La couche de backing 223 n'est par exemple pas découpée en secteurs.

La structure d'interconnexion 230 comprend une portion annulaire 232 prise entre la couche de backing 223 et la couche piézoélectrique 221, et reliée aux limandes 231.

Les limandes 231 sont repliées sur l'embout 203 et le fourreau 215.

Comme illustré dans les figures 3C et 3D, la portion annulaire 232 comprend des plots de contact électrique 234 reliés chacun à une piste métallique 236. Généralement, un plot de contact 234 est relié à un transducteur 225. Les pistes métalliques 236 se prolongent dans les limandes 231. Les plots de contact 234 et les pistes métalliques 236 sont par exemple dédiés aux signaux des transducteurs. Les pistes métalliques 236 sont isolées les unes des autres, et disposées dans, et/ou sur, un support isolant 237, ou support diélectrique. Le support diélectrique 237 est par exemple sous la forme d'un film de matériau polymère, de préférence flexible, par exemple en polyimide. Plusieurs autres matériaux peuvent convenir pour un support diélectrique flexible, par exemple un polyester, un polynaphtalate d'éthylène, un polyétherimide. Les pistes métalliques 236 peuvent avantageusement être en un matériau malléable, par exemple en or ou en cuivre. En effet, les pistes métalliques 236 sont repliées, en même temps que les limandes 231. La structure d'interconnexion 230 comprend en outre des bandes internes 233, ou pattes 233. Chaque patte 233 comprend un plot de contact électrique 235, qui est par exemple un plot de masse.

Les pattes 233 sont repliées à l'intérieur de la gaine 200, c'est-à-dire dans la lumière axiale 204, par exemple en contact avec la paroi interne de la bague distale 201.

La portion annulaire 232 est reliée aux limandes 231 et aux pattes 233, et est comprise entre les limandes 231 et les pattes 233 qui s'étendent radialement dans deux sens opposés les unes des autres à partir de la portion annulaire 232.

Par exemple, le nombre de pattes 233 est égal au nombre de limandes 231.

Selon un exemple de réalisation, la structure d'interconnexion 230 comprend seize limandes 231, et dix-huit pistes métalliques 236 par limande (deux pour la masse et seize connectées aux électrodes des éléments transducteurs de la sonde ultrasonore 220), ce qui permet de relier électriquement 256 transducteurs de manière indépendante.

Les pistes métalliques 236 ont par exemple une largeur d'environ 20 µm et sont espacées entre elles d'environ 50 µm, avec une largeur de limande d'environ 2 mm. On peut réaliser des pistes métalliques plus fines et plus resserrées entre elles. Par exemple, les pistes métalliques 236 peuvent avoir une largeur inférieure à 15 µm, par exemple égale à environ 5 µm, et un espacement inférieur à 25 µm, par exemple égal à environ 5 µm. Cela peut permettre de relier électriquement plus de transducteurs, par exemple plus que les 256 transducteurs indiqués dans l'exemple de la structure d'interconnexion 230.

Selon une autre solution pour pouvoir relier électriquement plus de transducteurs, qui peut être combinée avec la solution précédente, la structure d'interconnexion 230 peut comprendre plusieurs couches d'interconnexion, sur et/ou dans le support diélectrique 237, chaque couche d'interconnexion pouvant être similaire à celle décrite ci-dessus, et les pistes métalliques des différentes couches d'interconnexion pouvant être reliées entre elles par des connexions verticales appelées "vias". Ceci permet d'interconnecter un nombre très élevé de transducteurs, typiquement supérieur à 256, par exemple 512 transducteurs pour deux couches d'interconnexion similaires à celle décrite ci-dessus, 768 transducteurs pour trois couches d'interconnexion similaires à celle décrite ci-dessus, 1024 transducteurs pour quatre couches d'interconnexion similaires à celle décrite ci-dessus...

La structure d'interconnexion 230 peut être un circuit imprimé flexible, ou "FPCB", pour flexible printed circuit board en anglais.

La figure 3B montre que l'enveloppe externe 207 peut être prolongée sur la sonde 220 (portion 207A), comme décrit plus avant, et qu'en outre elle peut être prolongée à l'intérieur de la lumière axiale 204, en contact avec la paroi interne de la gaine 200.

La figure 4 est une vue schématique représentant un exemple de sonde d'imagerie ultrasonore 420 d'une gaine d'introduction médicale selon un mode de réalisation.

La sonde d'imagerie ultrasonore 420 peut correspondre à la sonde d'imagerie ultrasonore 220 des figures 2A, 2B et 3A. La gaine d'introduction médicale peut correspondre à la gaine 200 des figures 2A à 2C.

Dans la sonde d'imagerie ultrasonore 420, le réseau de transducteurs comprend plusieurs couronnes concentriques autour de la lumière axiale 204, chaque couronne comprenant chacune plusieurs transducteurs 425. Dans cet exemple, le réseau de transducteurs comprend le même nombre de transducteurs pour toutes les couronnes, une surface sensiblement égale pour tous les transducteurs, tout en ayant un écart sensiblement constant entre les transducteurs. Dans l'exemple représenté, le réseau de transducteurs comprend huit couronnes, et 128 transducteurs par couronne, formant une matrice de 1024 transducteurs.

Dans la sonde d'imagerie ultrasonore 420, une première électrode de chaque transducteur 425 est connectée individuellement à une piste conductrice de la structure d'interconnexion 230 et une seconde électrode est reliée à une masse commune avec les autres secondes électrodes des autres transducteurs de la sonde 420, de sorte que chaque transducteur peut être piloté individuellement.

A titre de variante, les premières électrodes des transducteurs 425 d'un même secteur angulaire peuvent être reliées entre elles, et les secondes électrodes des transducteurs d'une même couronne être également reliées entre elles. On obtient ainsi une matrice de transducteurs pilotable en rayon-angle dont le principe de pilotage est similaire aux matrices adressables en ligne-colonne (ou matrice RCA, de l'anglais Row-Column Addressed). Cette variante a pour avantage de réduire le nombre de connexions nécessaires pour un nombre de transducteurs équivalent, ou bien d'augmenter le nombre de transducteurs pour un nombre de connexions équivalent. Dans l'exemple de la figure 4, la sonde 420 nécessiterait par exemple alors 8 plus 128, soit 136 connexions au lieu de 1024.

Les exemples de réalisation décrits montrent qu'on peut disposer d'une gaine d'introduction médicale adaptée à recevoir un cathéter ou tout autre appareil médical allongé et qui comprenne une fonctionnalité d'imagerie. En outre, la gaine d'introduction médicale est orientable tout en permettant une connexion électrique des transducteurs ultrasonores.

La gaine d'introduction médicale selon les modes de réalisation peut trouver des applications dans le domaine du traitement de pathologies cardiaques, par exemple pour implanter un stimulateur cardiaque ("pacemarker" en anglais), pour réaliser une ablation par radiofréquence (RF ablation), ou pour remplacer ou implanter une valve cardiaque, par exemple réaliser une implantation de valve aortique transcathéter (TAVI, de l'anglais "Transcatheter Aortic Valve Implantation"), ou un remplacement de valve aortique transcathéter (TAVR, de l'anglais "Transcatheter Aortic Valve Replacement"), la gaine étant généralement utilisée en association avec un cathéter ou tout autre appareil médical allongé positionné dans la lumière axiale de la gaine. D'autres applications peuvent être envisagées, qui mettent en œuvre la gaine d'introduction médicale dotée d'une sonde d'imagerie ultrasonore selon les modes de réalisation, en association avec un appareil médical allongé du type cathéter.

Divers modes de réalisation et variantes ont été décrits. La personne du métier comprendra que certaines caractéristiques de ces divers modes de réalisation et variantes pourraient être combinées, et d'autres variantes apparaîtront à la personne du métier.

Enfin, la mise en oeuvre pratique des modes de réalisation et variantes décrits est à la portée de la personne du métier à partir des indications fonctionnelles données ci-dessus.

## Revendications

1. Gaine d'introduction médicale (100 ; 200) s'étendant selon une direction axiale (X) entre une extrémité distale (100A ; 200A) et une extrémité proximale (100B ; 200B) opposée à l'extrémité distale, la gaine d'introduction médicale comportant une lumière axiale (104 ; 204) interne entre ladite extrémité distale et ladite extrémité proximale, et une portion fléchissable (110 ; 210) s'étendant jusqu'à ladite extrémité distale, la portion fléchissable comprenant :
- une sonde d'imagerie ultrasonore (120 ; 220 ; 420) comprenant plusieurs transducteurs à ultrasons (225 ; 425) répartis en matrice sur une face transversale (200C) de la gaine d'introduction médicale à l'extrémité distale (200A), autour de la lumière axiale (104 ; 204) ;
- un coupleur d'orientation (211) adapté à conférer une courbure à la portion fléchissable (210) ;
- un fourreau (215) disposé autour du coupleur d'orientation (211), ledit fourreau comprenant une portion tubulaire (216) et une portion périphérique (217) protubérante enroulée en plusieurs tours en saillie (219) autour de la portion tubulaire (216), le long de la direction axiale (X), la portion tubulaire (216) étant comprise entre la portion périphérique (217) et le coupleur d'orientation (211) ; et
- des bandes de connexion (231) s'étendant depuis les transducteurs à ultrasons (225 ; 425) dans la direction axiale (X) en direction de l'extrémité proximale (200B) et réparties autour du fourreau (215) ; et
- une structure élastique (218) enroulée en plusieurs tours autour du fourreau (215) et des bandes de connexion (231), la structure élastique (218) cheminant entre les tours en saillie (219).

2. Gaine d'introduction médicale (200) selon la revendication 1, dans laquelle la portion périphérique (217) présente une forme en hélice, les tours en saillie (219) correspondant aux tours de l'hélice, la structure élastique (218) présentant également une forme en hélice cheminant entre les tours en saillie (219).

3. Gaine d'introduction médicale selon la revendication 1, dans laquelle les tours en saillie de la portion périphérique sont des anneaux en saillie disjoints entre eux, et répartis, par exemple régulièrement répartis, sur plusieurs circonférences de la portion tubulaire le long de la direction axiale, les tours de la structure élastique étant également des anneaux disjoints entre eux et positionnés entre les anneaux en saillie.

4. Gaine d'introduction médicale (200) selon l'une quelconque des revendications 1 à 3, dans laquelle le pas entre les tours de la structure élastique (218) est sensiblement égal au pas entre les tours en saillie (219) de la portion périphérique (217), les tours de la structure élastique étant répartis de manière régulière le long de la direction axiale (X).

5. Gaine d'introduction médicale selon l'une quelconque des revendications 1 à 3, dans laquelle le pas entre les tours de la structure élastique est sensiblement égal au pas entre les tours en saillie de la portion périphérique, les tours de la structure élastique étant répartis de manière irrégulière le long de la direction axiale (X), par exemple avec une plus grande densité autour d'un centre (C) de la portion fléchissable (210).

6. Gaine d'introduction médicale selon l'une quelconque des revendications 1 à 5, dans laquelle la lumière axiale est dimensionnée pour recevoir de manière coulissante et/ou rotative un cathéter.

7. Gaine d'introduction médicale (200) selon l'une quelconque des revendications 1 à 6, dans laquelle le coupleur d'orientation (211) comprend des maillons (212), par exemple des rotules, deux maillons adjacents coopérant l'un avec l'autre de manière à pouvoir pivoter autour d'au moins un axe de rotation.

8. Gaine d'introduction médicale (200) selon la revendication 7, dans laquelle le coupleur d'orientation (211) comprend des câbles de tringlerie (205, 206) reliés aux maillons (212) de manière à maintenir les maillons les uns contre les autres, et à contrôler les pivotements des maillons (212), et ainsi une flexion de la portion fléchissable (210).

9. Gaine d'introduction médicale (200) selon l'une quelconque des revendications 1 à 8, dans laquelle les transducteurs à ultrasons (225 ; 425) de la sonde ultrasonore (220 ; 420) sont répartis selon plusieurs couronnes concentriques autour de la lumière axiale (204), chaque couronne comprenant chacune plusieurs transducteurs répartis en secteurs radiaux le long de ladite couronne, par exemple le nombre de couronnes est supérieur à 3 et le nombre de transducteurs par couronne est supérieur à 30.

10. Gaine d'introduction médicale (200) selon l'une quelconque des revendications 1 à 9, dans laquelle les transducteurs à ultrasons (225) sont des transducteurs piézoélectriques et sont formés par plusieurs couches annulaires s'étendant l'une sur l'autre autour de la lumière axiale (204), lesdites couches annulaires comprenant :
- une couche piézoélectrique (221) métallisée sur chacune de ses faces interne et externe, et divisée en plusieurs secteurs piézoélectriques, par exemple des secteurs annulaires et radiaux ;
- une couche d'adaptation d'impédance (222) sur la couche piézoélectrique (221) et divisée en plusieurs secteurs d'adaptation d'impédance, chaque secteur d'adaptation d'impédance étant positionné en vis-à-vis d'un secteur piézoélectrique ; et
- une couche d'atténuation acoustique (223), sous la couche piézoélectrique (221).

11. Gaine d'introduction médicale selon la revendication 10, comprenant en outre une structure d'interconnexion (230) incluant les bandes de connexion (231), la structure d'interconnexion comprenant en outre une portion annulaire (232) disposée entre la couche d'atténuation acoustique (223) et la couche piézoélectrique (221), ladite portion annulaire comprenant des pistes métalliques (236) reliées aux transducteurs à ultrasons (225) et se prolongeant dans les bandes de connexion (231).

12. Gaine d'introduction médicale (200) selon l'une quelconque des revendications 1 à 11, comprenant en outre un embout (203) annulaire à l'extrémité distale (200A), ledit embout étant relié au coupleur d'orientation (211), par exemple à un maillon distal (212A) dudit coupleur d'orientation, les transducteurs à ultrasons (225) étant disposés dans une gorge circonférentielle (203C) dudit embout tout autour de la lumière axiale (204).

13. Gaine d'introduction médicale selon l'une quelconque des revendications 1 à 12, comprenant en outre une enveloppe externe (207) autour des bandes de connexion (231), du fourreau (215) et de la structure élastique (218), l'enveloppe externe étant par exemple en un matériau biocompatible.

14. Dispositif (10) d'exploration et d'intervention intracorporelles comprenant une gaine d'introduction médicale (100 ; 200) selon l'une quelconque des revendications 1 à 13.

15. Dispositif (10) selon la revendication 14, comprenant en outre :
- un cathéter configuré pour être introduit dans la lumière axiale (104 ; 204) de la gaine d'introduction médicale (100 ; 200) ; et/ou
- une poignée de contrôle (12) à l'extrémité proximale (100B ; 200B), adaptée à contrôler une flexion de la portion fléchissable (110 ; 210).
